(19) Europäisches Patentamt
European Patent Office
Office européen des brevets

(11) Veröffentlichungsnummer: **0 028 724**
**B1**

# (12) EUROPÄISCHE PATENTSCHRIFT

(45) Veröffentlichungstag der Patentschrift: **12.09.84**

(51) Int. Cl.³: **C 07 C 118/00,** C 07 C 119/048

(21) Anmeldenummer: **80106299.3**

(22) Anmeldetag: **16.10.80**

(54) **Verfahren zur Herstellung von Arylisocyanaten durch thermische Spaltung von Arylurethanen.**

(30) Priorität: **20.10.79 DE 2942543**

(43) Veröffentlichungstag der Anmeldung: **20.05.81 Patentblatt 81/20**

(45) Bekanntmachung des Hinweises auf die Patenterteilung: **12.09.84 Patentblatt 84/37**

(84) Benannte Vertragsstaaten: **BE DE FR GB IT NL**

(56) Entgegenhaltungen:
**DE - A - 2 635 490**
**US - A - 3 919 279**

(73) Patentinhaber: **BASF Aktiengesellschaft, Carl-Bosch-Strasse 38, D-6700 Ludwigshafen (DE)**

(72) Erfinder: **Merger, Franz, Dr., Max-Slevogt-Strasse 25, D-6710 Frankenthal (DE)**
Erfinder: **Towae, Friedrich, Dr., Parkstrasse 22, D-6700 Ludwigshafen (DE)**

**EP 0 028 724 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von Arylisocyanaten, wie z.B. Aryl-mono-, -di- und/oder -polyisocyanaten, durch thermische Spaltung von Arylurethanen bei Temperaturen von 175 bis 600 °C in Gegenwart von in heterogener Phase vorliegendem metallischem Zink oder Aluminium als Katalysatoren. Die Katalysatoren werden insbesondere in einer oberflächenreichen Form eingesetzt.

Es ist bekannt, dass N-substituierte Urethane thermisch in der Gasphase oder in flüssiger Phase in Isocyanate spaltbar sind. Bei der thermischen Spaltung treten gleichzeitig verschiedene unerwünschte Nebenreaktionen auf. Genannt seien beispielsweise die Decarboxylierungsreaktion der Urethane, die von der Bildung primärer und sekundärer Amine sowie Olefine begleitet sein kann; Umsetzungen zwischen dem gebildeten Isocyanat und Urethan zu Allophanaten bzw. Amin zu Harnstoffen und Polymerisation der Isocyanate zu Isocyanuraten.

Die Pyrolyse der Urethane in der Dampfphase – die Dampfphase ist hierbei so definiert, dass das Reaktionsgemisch, gegebenenfalls einschliesslich des Lösungsmittels, nach der Spaltung in der Dampfphase vorliegt, unabhängig davon, ob die zu spaltenden Urethane gasförmig, flüssig oder fest zugeführt werden – wird nach Angaben der DE-OS 1 944 719 (GB-PS 1 247 451) bei Temperaturen von 400 bis 600 °C in Gegenwart einer Lewissäure als Katalysator durchgeführt, wobei das Isocyanat und der Alkohol durch fraktionierte Kondensation getrennt wird. Toluylen-diisocyanat wird z.B. durch Pyrolyse von Toluylen-2,4-diäthylurethan in Gegenwart von Eisen-(III)-chlorid erhalten. Nachteile der Reaktion sind u.a. niedrige Ausbeuten, verbunden mit beträchtlichen Mengen eines polymeren Nebenprodukts, Zersetzung des Katalysators und Korrosion der Reaktionsapparatur. In der DE-OS 2 410 505 (US 3 870 739) wird ein Verfahren beschrieben, bei dem das Urethan bei einer Temperatur von 350 bis 550 °C und einem Druck von weniger als dem (m + l)fachen des Isocyanatdampfdruckes in einr katalysatorfreien Pyrolysezone innerhalb von 15 Sekunden gespalten wird. Nachteilig an diesem Verfahren ist u.a., dass eine grosse für die endotherme Spaltung erforderliche Wärmemenge innerhalb einer sehr kurzen Zeit dem pulverförmigen Urethan zugeführt werden muss und ein als Nebenprodukt anfallendes festes Polymer und dessen Abtrennung die Durchführung eines kontinuierlichen Verfahrens erschwert.

Die thermische Spaltung von Urethanen in flüssiger Phase wird beispielsweise in den DE-AS 2 421 503 (US 3 962 302) und DE-AS 2 530 001 (US 3 919 280) beschrieben. Nach Angaben der DE-AS 2 421 503 werden die Urethane in einem inerten Lösungsmittel, wie Alkylbenzolen, lienaren und cyclischen Kohlenwasserstoffen und/oder Phthalsäureestern, gelöst und unter Normal- oder Überdruck bei 175 bis 350 °C gespalten. Die Isolierung und Trennung des erhaltenen Isocyanats und Alkohols erfolgt mit Hilfe des Lösungsmittels als Trägermittel und/oder unter Verwendung eines Inertgases als Trägermittel. Gemäss DE-AS 2 530 001 werden als Reaktionsmedium höhermolekulare, gegebenenfalls substituierte aliphatische, cycloaliphatische oder aromatische Kohlenwasserstoffe, Äther, Ester oder Ketone verwendet. Zur Trennung der Spaltprodukte wird nur die Destillation genannt, wobei über Kopf Isocyanat, Alkohol und Trägermaterial abdestilliert wird, während das Reaktionsmedium als Bodenfraktion zurückbleibt.

Die US-A-3 919 279 beschreibt die chemische Spaltung von Urethanen in Gegenwart von Schwermetallkatalysatoren aus der Reihe Molybdän, Vanadium, Mangan, Eisen, Kobalt, Chrom, Kupfer und Nickel sowie von Mischungen oder Kombinationen der Schwermetalle. Für die erfolgreiche Durchführung des Verfahrens ist jedoch wesentlich, dass die Katalysatoren unter den Reaktionsbedingungen im Reaktionsmedium löslich sind.

Zur Herstellung von aromatischen Isocyanaten werden die Urethane nach DE-OS 2 635 490 bei Temperaturen von 150 bis 350 °C unter vermindertem Druck mit einer Lösung aus wenigstens einem Metallion, wie Ionen von Kupfer, Zink, Aluminium, Zinn, Titan, Vanadium, Eisen, Kobalt und Nickel aus Katalysator, der in einem Lösungsmittel mit einem Siedepunkt von 200 °C in einer Metallkonzentration von wenigstens 0,001 Gew.-%, bezogen auf das Lösungsmittel, gelöst ist, in Kontakt gebracht. Die Trennung der erhaltenen Spaltprodukte erfolgt durch fraktionierte Kondensation. Nach den genannten Methoden können Urethane in Abhängigkeit von ihrer Struktur in teilweise sehr guten Ausbeuten in Isocyanate übergeführt werden. Nicht beispielhaft beschrieben wird in diesen Publikationen die Herstellung von Mischungen aus Diphenylmethan-diisocyanaten und Polyphenyl-polymethylen-polyisocyanaten (roh-MDI) aus den entsprechenden Urethanmischungen (roh-MDU). Im Unterschied zu den beschriebenen Isocyanaten ist roh-MDI nicht mit Hilfe von Lösungsmitteln als Träger vollständig destillierbar und kann daher nicht wie ausgeführt von Katalysator, Lösungsmittel und gegebenenfalls nicht umgesetzten Ausgangsstoffen sowie Verunreinigungen isoliert werden.

Aufgabe der vorliegenden Erfindung war es, die thermische Spaltung von Arylurethanen zu Arylisocyanaten, insbesondere von höhermolekularen Aryl-di- und/oder -polyurethanen zu Aryl-di-und/oder -polyisocyanaten, weiter zu verbessern, wobei die obenbeschriebenen Nachteile beseitigt werden sollten.

Diese Aufgabe wurde gelöst durch ein Verfahren zur Herstellung von Arylisocyanaten durch thermische Spaltung von Arylurethanen in Gegenwart eines Katalysators bei Temperaturen von 175 bis 600 °C, das dadurch gekennzeichnet ist, dass man als Katalysatoren metallisches Zink oder Aluminium verwendet, die in heterogener Phase vorliegen.

Die erfindungsgemäss verwendbaren Metalle sind nicht nur gute Wärmeüberträger, sondern sie haben darüber hinaus einen guten, technisch vorteilhaft verwertbaren Katalyseeffekt und ermöglichen deutliche Senkungen der Reaktionstemperaturen und/oder -zeiten, so dass Nebenreaktionen, wie Polymerisationen, eine geringere Rolle spielen. Durch eine vorzügliche Aktivität zeichnen sich besonders Zink und Aluminium aus. Bei der Spaltung von Aryl-diurethanen in der Gasphase erreicht mant z.B. mit der Zink- und Aluminium-Heterogenkatalyse für die gleiche Spaltgeschwindigkeit eine Senkung der Spalttemperatur von 50 bis 100 °C gegenüber bekannten Verfahren. Unter «Gasphase» im Sinne der Erfindung wird hierbei die vorher genannte Definition verstanden. Vorteilhaft ist ferner, dass die in heterogener Phase vorliegenden Metalle leicht aus dem Reaktionsgemisch abgetrennt und wiederverwendet werden können. Die Entfernung gelöster Katalysatoren aus den Reaktionsprodukten ist bekanntermassen problematisch, insbesondere wenn die Arylisocyanate nicht destillierbar sind.

Wie bereits dargelegt wurde, eignen sich zur thermischen Spaltung von Arylurethanen durch Heterogenkatalyse, die in der Gasphase oder in flüssiger Phase erfolgen kann, als Katalysatoren metallisches Zink oder Aluminium. Anstelle der Metalle können auch diese, gegebenenfalls in Kombination mit anderen Metallen, wie Vanadium und/oder Wolfram, enthaltende Legierungen eingesetzt werden. Die Katalysatoren, die erfindungsgemäss in heterogener Phase im Reaktionsgemisch vorliegen, werden vorzugsweise in einer oberflächenreichen Form, beispielsweise in Form von Metallwolle, -spänen, -pulver oder -granulaten mit durchschnittlichen Teilchendurchmessern von 1 bis 10 mm, vorzugsweise 2 bis 6 mm, verwendet. Die Katalysatoren können in verschiedenen Anordnungen angewandt werden, beispielsweise als Festbett, z.B. durch Beschicken von verschieden gestalteten Reaktoren, wie Rohr-, Tank- oder Kesselreaktoren mit Metallgranulaten, -ringen, -spänen oder -wolle, so dass das Reaktionsgemisch kontinuierlich durch das Katalysatorfestbett geleitet werden kann, oder auch in Suspensionen in Rührreaktoren.

Die erfindungsgemässen Katalysatoren können im Festbett in der Gasphase mit 0,1 bis 20, vorzugsweise 1 bis 10 Arylurethanäquivalenten/Liter Katalysator und Stunde und in flüssiger Phase mit 0,1 bis 5, vorzugsweise 0,2 bis 3 Arylurethanäquivalenten/Liter Katalysator und Stunde beaufschlagt werden. In Suspension sind teilweise höhere Belastungen als im Festbett möglich.

Als Lösungsmittel können gegebenenfalls solche verwendet werden, die gegenüber den Isocyanaten und übrigen Komponenten unter den Reaktionsbedingungen inert sind und sich im Siedepunkt von diesen unterscheiden. Lösungsmittel, deren Siedepunkt zwischen den Siedepunkten des Arylisocyanates und des abgespaltenen Alkohols liegen, sind zweckmässig für Spaltungen in flüssiger Phase, wenn das Arlyisocyanat als nicht destillierte Bondenfraktion isoliert wird; sie können aber auch bei Spaltungen in der Gasphase als Verdünnungsmittel Anwendung finden und dort gegebenenfalls die Isolierung des Isocyanats durch fraktionierende Kondensationen ohne Rekombination mit dem Alkohol erleichtern. Lösungsmittel, deren Siedepunkt höher liegt als die Siedepunkte der Spaltprodukte, werden bevorzugt für die Spaltung von Arylurethanen in flüssiger Phase zu Arylisocyanaten, die durch Destillation gegebenenfalls unter Mithilfe von Strippmitteln isoliert werden. Gegebenenfalls kann hierbei zu einer verbesserten Isolierung von Alkohol und Isocyanat zusätzlich ein zwischen Alkohol und Isocyanat siedendes Lösungsmittel eingesetzt werden. Besonders bewährt haben sich und vorzugsweise zur Anwendung kommen Dibenzylnaphthaline, die durch Benzylierung von Naphthalin und Benzylchlroid zugänglich sind, für Spaltungen von Di- und/oder Polyurethanen in flüssiger Phase zu Aryl-di- und/oder -polyisocyanaten, deren Siedepunkt tiefer liegt als der Siedepunkt des Dibenzylnaphthalins. Vorteilhaft ist, wenn die Arylurethane in dem Lösungsmittel löslich sind, obgleich dies nicht unbedingt notwendig ist. Das Lösungsmittel ist gleichzeitig Wärmemedium, das dazu dient, dem Reaktionssystem Wärme zuzuführen und die Reaktionstemperatur gleichmässig zu gestalten.

Als Lösungsmittel seien beispielhaft genannt: Aliphatische Kohlenwasserstoffe, wie die höheren Alkane Decan, Undecan, Dodecan, Tridecan, Tetradecan, Pentadecan, Hexadecan, Heptadecan, Dekalin, flüssiges Paraffin, Erdölfraktionen von Paraffinen, die üblicherweise als Schmieröle, Kühlöle oder Schneidöle verwendet werden; alicyclische Kohlenwasserstoffe, wie Erdölfraktionen der Naphthenreihen; gegebenenfalls substituierte aromatische Kohlenwasserstoffe, wie z.B. Naphthalin, 1- und 2-Methylnaphthalin, 1,2-, 1,4-, 1,6-, 2,7-, 2,6- und 2,3-Dimethylnaphthalin, 1-Äthylnaphthalin, Phenylnaphthalin, Benzylnaphthalin, Toluol, 1,2-, 1,3- und 1-4-Dimethylbenzol, 1,2,4- und 1,3,5-Trimethylbenzol, 1,2,3,5- und 1,2,4,5-Tetramethylbenzol, 1,3,5-Triäthylbenzol, Hexyl-, Heptyl-, Octyl-, Nonyl-, Decyl- und Dodecylbenzol, Hexamethylbenzol, Hexaäthylbenzol, Diphenyl, 4,4'-Dimethyldiphenyl, Dibenzyl, Diphenylmethan und 4,4'-Dimethyl-diphenylmethan, halogensubstituierte aromatische Kohlenwasserstoffe, wie z.B. Chlorbenzol, 1,2- und 1,4-Dichlorbenzol, 1,4-Dijodbenzol, 1,2,3- und 1,3,5-Trichlorbenzol, 1,2,3,4-, 1,2,3,5- und 1,2,4,5-Tetrachlorbenzol, Pentachlorbenzol, 1- und 2-Fluornaphthalin, 1- und 2-Chlornaphthalin, 1- und 2-Jodnaphthalin und Diphenyl-dichlormethan; nitrogruppenhaltige aromatische Kohlenwasserstoffe, wie z.B. Nitrobenzol, 3-Nitrotoluol, 2-Nitro-m-xylol, 5-Nitro-m-xylol und 4-Nitroanisol, aliphatische und aromatische Ketone, wie z.B. Cyclohexanon, Cycloheptanon, Di-n-butylketon, Di-n-amylketon, α-Tetralon, Acetophenon, Propiophenon, Benzophenon, 3-Methylbenzophenon, Dodecanon-2 und Tridecanon-2, Sulfone und Carbonsäureester, wie z.B. Sulfolan, Diäthylsulfon, Phthalsäuredimethylester, Phthalsäurediäthyl-

ester, Benzoesäurepropylester und Laurinsäureäthylester und Äther, wie z.B. Diäthylenglykol-dimethyläther, Diäthylenglykoldiäthyläther, Diisoamyläther, Di-n-amyläther, Resorcindimethyläther, Resorcindiäthyläther, Phenyloctyläther, Phenylbenzyläther, Dibenzyläther, Diphenyläther, α-Methylnaphthyläther und β-Äthylnaphthyläther.

Arylurethane, die nach dem erfindungsgemässen Verfahren thermisch in Arylisocyanate und Alkohole gespalten werden, besitzen die allgemeine Formel

Ar (NHCOOR)n

in der Ar einen Arylrest, R einen gegebenenfalls substituierten aliphatischen oder aromatischaliphatischen Rest mit 1 bis 20 Kohlenstoffatomen, vorzugsweise 1 bis 10 Kohlenstoffatomen oder einen cycloaliphatischen Rest mit 3 bis 15 Kohlenstoffatomen, vorzugsweise 3 bis 7 Kohlenstoffatomen und n eine ganze Zahl von 1 bis 8 und höher, vorzugsweise 2 bis 6 bedeuten.

Als Arylreste seien beispielhaft genannt, die Reste von aromatischen Monoaminen, wie Anilin, substituierten Anilinen, wie in 2-, 3- und/oder 4-Stellung durch eine Nitro-, Methyl-, Ethyl-, n-Propyl, Isopropyl-, n-Butyl-, Isobutyl-, sek.-Butyl-, tert.-Butylgruppe oder ein Chloratom substituierten Anilinen, ortho-, meta- und/oder para-Hydroxy-, Methoxy-, Äthoxy-, Propoxy-, Isopropoxy-, N-Butoxy-, Isobutoxy-, sek.-Butoxy- und tert.-Butoxyanilin; durch eine Aminogruppe in m- und/oder p-Stellung substituierte Benzoesäurealkylester mit 1 bis 4 Kohlenstoffatomen im Alkylrest, durch eine Aminogruppe in m- und/oder p-Stellung substituierte N-Alkoxycarbonylaminobenzole und -toluole mit 1 bis 4 Kohlenstoffatomen im Alkylrest; α- und β-Naphthylamin; aromatischen Diaminen wie 1,3- und 1,4-Diaminobenzol; durch eine Nitro-, Methyl-, Äthyl-, n-Propyl-, Isopropyl-, n-Butyl-, Isobutyl-, sek.-Butyl-, tert.-Butyl-, Methoxy-, Äthoxy-, n-Propoxy-, Isopropoxy-, n-Butoxy-, Isobutoxy-, sek.-Butoxy-, tert.-Butoxygruppe oder ein Halogenatom, vorzugsweise ein Fluor- und/oder Chloratom, in 2- und/oder 4-Stellung substituiertem 1,3-Diaminobenzol oder in 2-Stellung substituiertem 1,4-Diaminobenzol, 1,5- und 1,8-Diaminonaphthalin, 4,4′-Diaminodiphenyl, 2,2′-, 2,4′- und 4,4′-Diaminodiphenylmethan und den entsprechenden Isomerengemischen und aromatischen Polyaminen, wie 1,3,5-Triaminobenzol, 2,4,6-Triaminotoluol und 1,3,5-Triaminonaphthalin, Polyphenyl-polymethylen-polyaminen sowie Mischungen aus Diamino-diphenylmethanen und Polyphenyl-polymethylen-polyaminen, die nach bekannten Verfahren durch Kondensation von Anilin und Formaldehyd in Gegenwart von vorzugsweise Mineralsäuren als Katalysatoren erhalten werden.

Besonders bewährt haben sich und daher vorzugsweise verwendet werden als Arylreste die Reste von aromatischen Monoaminen, wie o-, m- und/oder p-Toluidin, o-, m- und/oder p-Anisidin, 3-Hydroxyanilin, o-, m- und/oder p-Chloranilin, 2,4-, 3,4- und 3,5-Dichloranilin, 2-Nitro-4-aminotoluol, 4-Nitro-2-aminotoluol, 2-Nitro-6-aminotoluol und N-Alkoxycarbonyl-arylaminen der Formel

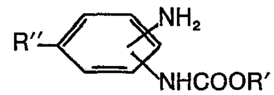

in der R′ einen Methyl-, Äthyl-, Propyl-, Isopropyl-, n-Butyl-, Isobutyl-, sek.-Butyl- oder tert.-Butylrest und R″ ein Wasserstoffatom oder den Rest R′ bedeuten sowie insbesondere Anilin; von aromatischen Diaminen wie 3,3′-Ditoluylen-4,4′-diamin, 2,4- und 2,6-Toluylendiamin sowie den entsprechenden Isomerengemischen, 2,2′-, 2,4′- und 4,4′-Diaminodiphenylmethan und den entsprechenden Isomerengemischen, 1,5- und 1,8-Naphthylen-diamin und von Polyaminen, wie Mischungen aus Diamino-diphenyl-methanen und Polyphenyl-polymethylen-polyaminen.

Als gegebenenfalls substituierte aliphatische, aromatisch-aliphatische oder cycloaliphatische Reste R kommen beispielsweise in Betracht: der Methyl-, Äthyl-, n-Propyl-, iso-Propyl-, n-Butyl-, iso-Butyl-, sek.-Butyl-, 2- und 3-Methylbutyl-, Neopentyl-, Pentyl-, 2-Methylpentyl-, sek.-iso-Amyl-, n-Hexyl-, 2-Äthylhexyl-, Heptyl-, n-Octyl-, n-Nonyl-, n-Decyl-, n-Dodecyl-, 2-Phenylpropyl-, Benzyl-, Cyclopentyl-, Cyclohexyl-, tert.-Butylcyclohexyl- und Bicyclo-(2,2,1)-heptylrest. Vorzugsweise verwendet wird als Rest R der Methyl-, Äthyl-, Propyl-, Butyl-, iso-Butyl-, 2- und 3-Methylbutyl-, Pentyl-, n-Hexyl-, 2-Äthylhexyl-, n-Heptyl-, n-Octyl- und Cyclohexylrest.

Als typische Beispiele für Arylurethane, die nach dem erfindungsgemässen Verfahren thermisch spaltbar sind, seinen genannt: N-Phenylmethylurethan, N-Phenyl-äthylurethan, 3,5-Dichlorphenyl-äthylurethan, 4-Methylphenyl-äthylurethan, 2,4- und 2,6-Toluylen-di-methylurethan sowie die entsprechenden Isomerengemische, 2,4- und 2,6-Toluylen-diäthylurethan, 2,4- und 2,6-Toluylen-di-butylurethan, 1,5-Naphthylen-di-äthylurethan, 4,4′-, 2,4′- und 2,2′-Methylendiphenyl-dimethylurethan, 4,4′-, 2,4′- und 2,2′-Methylen-diphenyl-diäthyluretnan, 4,4′-, 2,4′-, 2,2′-Methylen-diphenyl-dibutyl-urethan, 4,4′-, 2,4′-, 2,2′-Methylen-diphenyl-di-hexylurethan sowie die entsprechenden Isomerengemische und Mischungen aus 4,4′-, 2,4′-, 2,2′-Methylen-diphenyl- und Polymethylen-polyphenyl-di- und poly-methylurethan, -äthylurethan, -butylurethan, und -hexylurethan.

Die thermische Spaltung der Arylurethane kann sowohl in der Gasphase bei Temperaturen von 300 bis 600 °C, vorzugsweise 330 bis 380 °C als auch in flüssiger Phase bei Temperaturen von 175 bis 350 °C, vorzugsweise 220 bis 320 °C diskontinuierlich oder kontinuierlich bei vermindertem normalen oder erhöhtem Druck, beispielsweise in der Gasphase bei Drücken von 1 mbar bis 1 bar, vorzugsweise 1 bis 100 mbar, durchgeführt werden. Die Spaltung der Arylurethane und die Trennung der Spaltprodukte durch Abdestillieren des Alkohols und gegebenenfalls des Arylisocyanats

und/oder des Lösungsmittels können gleichzeitig oder nacheinander erfolgen. In der Gasphase spaltet man in der Regel unter vermindertem Druck, dessen Maximum bei vorgegebener Temperatur gleich ist dem Siededruck des Reaktionsgemisches. In flüssiger Phase wählt man bei simultaner Spaltung und Trennung zweckmässig ein Temperatur/Druck-Verhältnis, das dem Siedepunkt der tiefstsiedenden Komponente der Bodenfraktion entspricht. In geschlossenem System, z.B. einem Rohrspaltrekator, erfolgt die Spaltung zweckmässig ohne Erhöhung des Systemdrucks.

Man kann die Ausgangsstoffe dampfförmig, geschmolzen oder in fester Form, z.B. als Pulver, als Suspension oder als Lösung, in einem inerten Lösungsmittel dem Reaktor zuführen, der wahlweise auf einer bestimmten Temperatur und einem bestimmten Druck gehalten wird. Man führt z.B. ein Aryldiurethan – dampfförmig, flüssig oder fest – entsprechend 0,1 bis 20, vorzugsweise 1 bis 10 Urethanäquivalente pro Liter und Stunde bei einer Temperatur von 300 bis 400 °C, vorzugsweise von 330 bis 380 °C, und einem Druck von 1 mbar bis 1 bar, vorzugsweise von 1 bis 100 mbar, in einen mit Zinkspänen beschickten Rohrreaktor und kondensiert das Aryl-diisocyanat, den Alkohol und gegebenenfalls nicht umgesetzte Aryldiurethan zweckmässig unter Zufuhr eines inerten Lösungsmittels, dessen Siedepunkt zwischen den Siedepunkten des Alkohols und des Isocyanates liegt, über eine Kolonne fraktionierend. Man kann die Lösung eines Di- und/oder Polyurethans in einem inerten Lösungsmittel bei einer Temperatur von 175 bis 350 °C, vorzugsweise 220 ibs 320 °C durch einen mit Zink- oder Aluminiumgranulaten beschickten Rohrreaktor und das Produkt anschliessend zur Trennung in eine Kolonnen oder durch eine Kaskade aus mehreren Spaltrekatoren und Trennkolonnen in abwechselnder Anordnung leiten. In einer anderen bevorzugten Ausführungsform führt man die Lösung kontinuierlich in einen Spaltreaktor bzw. eine Reaktorkaskade unter gleichzeitiger Abtrennung des Alkohols, gegebenenfalls mit Hilfe eines Strippmittels, z.B. eines Inertgases oder Zwischensieders, und des Arylisocyanates über eine oder mehrere Trennkolonnen unter Rückfluss des Lösungsmittels, das als Bodenfraktion abgezogen wird. In einer weiteren bevorzugten Verfahrensvariante kann man das Aryl-di- und/oder -polyurethan, z.B. roh-MDU, in einem geeigneten Lösungsmittel wie oben beschrieben in flüssiger Phase spalten, gleichzeitig den Alkohol unter Rückfluss des Lösungsmittels abdestillieren und anschliessend aus dem Sumpf das Lösungsmittel durch schonende Destillation, gegebenenfalls unter Strippen mit kurzer Verweilzeit, unter Rückfluss des Aryl-di- und/oder -polyisocyanates abdestillieren und das Aryl-di- und/oder -polyisocyanat als Bodenfraktion abführen. Hierbei hat es sich als vorteilhaft gezeigt, einen Anteil, beispielsweise 5 bis 10 Gew.-% des Aryldiisocyanates, Diphenylmethan-di-isocyanat mit dem Lösungsmittel abzudestillieren und mit diesem zusammen im Kreislauf in den Spaltreaktor zurückzuführen.

Nach dem erfindungsgemässen Verfahren können Arylisocyanate mit hoher Reinheit in grossen Ausbeuten wirtschaftlich vorteilhaft hergestellt werden. Die Vorteile des erfindungsgemässen Verfahrens werden besonders deutlich am Beispiel des roh-MDI, dessen thermische Spaltung bislang nicht beispielhaft beschrieben ist.

Die erfindungsgemäss erhaltenen Arylmonoisocyanate sind wertvolle Zwischenprodukte zur Herstellung von Schädlingsbekämpfungsmitteln, Farbstoffen und Hilfsmitteln; die Aryl-di- und/oder -polyisocyanate werden vorzugsweise zur Herstellung von Polyurethan-Kunststoffen verwendet.

Beispiel 1

20 Teile N-Phenylmethylurethan werden in 30 Teilen Dibenzylnaphthalin gelöst und mit einer Zulaufrate von 320 Litern pro Liter Reaktionsraum und Stunde in einen mit 5 Teilen Aluminiumgranulat einer Korngrösse von 1–3 mm Durchmesser gefüllten, auf 330 °C erhitzten Rührspaltrekator mit einem Volumen von 150 ml mit Überlauf aus Quarzglas eingeführt. An den Spaltreaktor sind zur Gewinnung der gasförmig abzuziehenden Produkte zwei Kühlfallen angeschlossen. In der ersten, wassergekühlten Vorlage kondensieren 6,9 Teile Phenylisocyanat und in der nachgeschalteten, mit Trockeneis gekühlten Vorlage bei der Spaltung gebildetes Methanol. Gaschromatographisch (nach der Methode des «Internen Standard») werden im Spaltlösungsmittel weitere 7,3 Teile Phenylisocyanat nachgewiesen; demnach sind insgesamt 14,2 Teile Phenylisocyanat (97,4% der Theorie, bezogen auf umgesetztes Phenylurethan) entstanden. Im Spaltlösungsmittel werden gaschromatographisch noch 1,5 Teile Ausgangsprodukt nachgewiesen, so dass der Umsatz an Phenylmethylurethan 92,5% beträgt.

Vergleichsbeispiel

Man verfährt analog Beispiel 1, beschickt den Spaltreaktor jedoch nicht mit einem Katalysator.

Hierbei zeigt sich, dass nur 13,3% des eingesetzten Phenylmethylurethans umgesetzt werden, wobei 2,0 Teile Phenylisocyanat (95,4% der Theorie, bezogen auf umgesetztes Phenylmethylurethan) erhalten werden.

Beispiele 2 bis 4

Man verfährt analog den Angaben von Beispiel 1, variiert jedoch das Katalysatormaterial und die eingesetzten Arylurethane.

Die Ergebnisse sind in der Tabelle aufgeführt.

Beispiel 5

86 Teile 2,4-Di-(methoxicarbonylamino)-toluol werden über eine Pulverdosiereinrichtung mit einer Rate von ca. 500 Litern pro Liter Reaktionsraum und Stunde in einen auf 350 °C erhitzten, mit Zinkspänen gefüllten Rohrreaktor aus Quarzglas kontinuierlich eingeführt. Im Spaltreaktor wird ein Druck von 10–15 mbar aufrechterhalten. Die austretenden Spaltgase werden fraktioniert kondensiert, wobei in einer mit Wasser gekühlten Vorlage 61 Teile Toluylendiisocyanat (TDI) (97,0% der

Tabelle

| Beispiel Nr. | Aryl-urethan | Katalysator | Umsatz Urethan % | Ausbeute Isocyanat % |
|---|---|---|---|---|
| 2 | 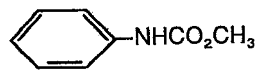 | Zink | 92,8 | 98,6 |
| 3 | 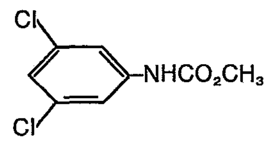 | Aluminium | 97,3 | 99,4 |
| 4 | 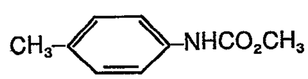 | Zink | 77,1 | 89,9 |

Theorie, bezogen auf eingesetztes 2,4-Di-(methoxicarbonylamino)-toluol) erhalten werden.

Beispiel 6

80 Teile 4,4'-Di-(hexoxicarbonylamino)-diphenylmethan werden in 80 Teilen 1,2,4,5-Tetramethylbenzol gelöst. Diese Lösung gibt man mit einer Zulaufrate von 100 Litern pro Liter Reaktionsraum und Stunde in einen auf 350 °C erhitzten, mit Aluminiumspänen gefüllten Rohrreaktor aus Quarzglas, in dem ein Druck von 5–10 mbar aufrechterhalten wird. Das bei der Spaltung gebildete Hexanol wird gasförmig abgetrennt und mit verdampftem Tetramethylbenzol in einer mit Wasser gekühlten Vorlage kondensiert. Als Reaktoraustrag erhält man 65 Teile einer Lösung von 32 Teilen Methylendiphenyldiisocyanat (72,6% der Theorie, bezogen auf umgesetztes 4,4'-Di-(hexoxicarbonylamino)-diphenylmethan) und 16 Teilen 4-(Hexoxicarbonylamino)-4-isocyanato-diphenylmethan in Tetramethylbenzol. Der Umsatz an 4,4'-Di-(hexoxicarbonylamino)-diphenylmethan ist quantitativ.

Beispiel 7

25 Teile 1,5-Di-(äthoxicarbonylamino)-naphthalin werden über eine Pulverdosiereinrichtung mit einer Rate von 450 Litern pro Liter Rekationsraum und Stunde in einen auf 350 °C erhitzten, mit Zinkspänen gefüllten Rohrreaktor eingeführt. Im Spaltreaktor wird ein Druck von 1–3 mbar aufrechterhalten. Die austretenden Spaltgase werden fraktioniert kondensiert, wobei in einer ersten mit Wasser gekühlten Vorlage 14 Teile 1,5-Naphthylendiisocyanat (NDI) (80,5% der Theorie, bezogen auf eingesetztes Urethan) vom Schmp. 129–132 °C erhalten werden.

Beispiel 8

170 Teile 2,4-Di-(butoxicarbonylamino)-toluol werden in 350 Teilen Dodecylbenzol gelöst. Diese Lösung gibt man mit einer Zulaufrate von 300 Litern pro Liter Reaktionsraum und Stunde in einen auf 320 °C erhitzten, mit Aluminiumgranulat der Korngrösse 1–3 mm gefüllten Spaltreaktor aus Quarzglas. Das bei der Reaktion gebildete Butanol wird unter Verwendung von 8 Litern Stickstoff je Liter Reaktionsgemisch und Stunde als Schleppmittel gasförmig abgetrennt und in einer mit Trockeneis gekühlten Vorlage kondensiert. Man erhält 443 Teile Reaktionsaustrag, aus dem durch Destillation bei 76–82 °C und 0,2 mbar 81 Teile 2,4-Toluylendiisocyanat (88,2% der Theorie, bezogen auf umgesetztes 2,4-Di-(butoxycarbonylamino)-toluol) erhalten werden. Das Ausgangsprodukt hat sich vollständig umgesetzt, im Destillationsrückstand wird gaschromatographisch noch ein Gemisch von rückführbarem 2-Butoxicarbonylamino-4-isocyanatotoluol und 4-(Butoxicarbonylamino)-2-isocyanatotoluol nachgewiesen.

Beispiel 9

Man setzt 10 Teile eines handelsüblichen «Roh-MDI»-Gemischs mit Hexanol zu einem Gemisch von Methylen-diphenyl-dihexylurethanen und Polymethylen-polyphenyl-polyhexylurethanen um und bezeichnet dieses Gemisch als «Vergleichsprodukt». Nun setzt man weitere 100 Teile desselben handelsüblichen «Roh-MDI»-Gemischs mit Methanol zu einem Gemisch von Methylen-diphenyl-dimethylurethanen und Polymethylen-polyphenyl-polymethylurethanen um. 120 Teile dieses Urethangemischs löst man in 400 Teilen Decylbenzol und gibt diese Lösung mit einer Zulaufrate von 300 Litern pro Liter Reaktionsraum und Stunde in einen auf 320 °C erhitzten, mit Zinkspänen gefüllten Rohrspaltrekator aus Quarzglas. Das bei der Spaltung gebildete Methanol wird gasförmig abgetrennt und in einer mit Trockeneis gekühlten Vorlage kondensiert. Man erhält 510 Teile Reaktionsaustrag, aus dem man das Lösungsmittel weitgehend abdestilliert, und den man mit 100 Teilen Hexanol versetzt. Man erhält so eine Lösung von Methylen-diphenyl-dihexylurethanen und Polymethylen-poly-phenyl-polyhexylurethanen, deren Hochdruck-Flüssigchromatogramm identisch ist mit dem des «Vergleichsprodukts».

## Patentansprüche

1. Verfahren zur Herstellung von Arylisocyanaten durch thermische Spaltung von Arylurethanen in Gegenwart eines Katalysators bei Temperaturen von 175 bis 600 °C, dadurch gekennzeichnet, dass man als Katalysatoren metallisches Zink oder Aluminium verwendet, das in heterogener Phase vorliegt.

2. Verfahren nach Anspruch 1, dadurch gekennzeichnet, dass man die Arylurethane in flüssiger Phase in einem Lösungsmittel bei Temperaturen von 175 bis 350 °C in Gegenwart von in heterogener Phase vorliegendem metallischem Zink oder Aluminium als Katalysatoren thermisch spaltet.

3. Verfahren nach Ansprüchen 1 bis 2, dadurch gekennzeichnet, dass man als Katalysatoren Zink oder Aluminium verwendet, die in der heterogenen Phase in einer oberflächenreichen Form vorliegen.

4. Verfahren nach Anspruch 1, dadurch gekennzeichnet, dass man die Arylurethane in der Gasphase bei Temperaturen von 300 bis 600 °C in Gegenwart von in heterogener Phase vorliegendem metallischem Zink oder Aluminium als Katalysator thermisch spaltet.

5. Verfahren nach Ansprüchen 1 und 4, dadurch gekennzeichnet, dass das Zink oder Aluminium in der heterogenen Phase in einer oberflächenreichen Form vorliegt.

## Claims

1. A process for the preparation of aryl isocyanates by thermal decomposition of aryl urethanes in the presence of a catalyst at a temperature of from 175 to 600 °C, wherein metallic zinc or aluminium which is present in the heterogeneous phase, is used as catalyst.

2. A process as claimed in claim 1, wherein the aryl urethanes are thermally decomposed in the liquid phase in a solvent at a temperature of from 175 to 350 °C in the presence of metallic zinc or aluminium, present in the heterogeneous phase, as catalyst.

3. A process as claimed in claim 1 or 2, wherein zinc or aluminium, which is present in the heterogeneous phase in a form having a large surface area, is used as catalyst.

4. A process as claimed in claim 1, wherein the aryl urethanes are thermally decomposed in the gas phase at a temperature of from 300 to 600 °C in the presence of metallic zinc or aluminium, present in the heterogeneous phase, as catalyst.

5. A process as claimed in claims 1 and 4, wherein the zinc or aluminium is present in the heterogeneous phase in a form having a large surface area.

## Revendications

1. Procédé de préparation d'arylisocyanates par décomposition thermique d'aryluréthanes en présence d'un catalyseur, à des températures de 175 à 600 °C, caractérisé par le fait qu'on utilise, comme catalyseurs, du zinc ou aluminium métalliques qui se trouvent en phase hétérogène.

2. Procédé selon la revendication 1, caractérisé par le fait qu'on décompose thermiquement les aryluréthanes en phase liquide dans un solvant, à des températures de 175 à 350 °C, en présence, comme catalyseurs, de zinc ou aluminium métalliques se trouvant en phase hétérogène.

3. Procédé selon les revendications 1 et 2, caractérisé par le fait qu'on utilise, comme catalyseurs, du zinc ou aluminium qui se trouvent sous une forme riche en surface dans la phase hétérogène.

4. Procédé selon la revendication 1, caractérisé par le fait qu'on décompose thermiquement les aryluréthanes en phase gazeuse, à des températures de 300 à 600 °C, en présence, comme catalyseurs, de zinc ou aluminium métalliques se trouvant en phase hétérogène.

5. Procédé selon les revendications 1 et 4, caractérisé par le fait que le zinc ou l'aluminium se trouvent sous une forme riche en surface, dans la phase hétérogène.